# EUROPÄISCHE PATENTSCHRIFT

(11) **EP 1 135 527 B1**
(45) Veröffentlichungstag und Bekanntmachung des Hinweises auf die Patenterteilung: **16.10.2002**
(21) Anmeldenummer: 99962118.8
(22) Anmeldetag: 26.11.1999
(51) Int. Cl.: C12Q 1/68, B01J 19/00

(54) **KOPIEREN UND KLONIEREN AN OBERFLÄCHEN**
CLONING AND COPYING ON SURFACES
CLONAGE ET COPIE SUR DES SURFACES

(30) Priorität: 27.11.1998 DE 19854946
(43) Veröffentlichungstag der Anmeldung: 26.09.2001
(73) Patentinhaber: Noxxon Pharma AG, 13355 Berlin (DE); Von Kiedrowski, Günter, 44797 Bochum-Stiepel (DE)
(72) Erfinder: VON KIEDROWSKI, Günter, D-44797 Bochum (DE); FÜRSTE, Jens, Peter, D-13559 Berlin (DE); KLUSSMANN, Sven, D-10709 Berlin (DE); KLEIN, Thomas, D-14109 Berlin (DE)
(74) Vertreter: Omsels, Hermann-Josef
(86) Internationale Anmeldenummer: DE9903856
(87) Internationale Veröffentlichungsnummer: WO00032809

(56) Entgegenhaltungen:
- EP-A- 0 374 665
- WO-A-00/27521
- WO-A-93/17126
- WO-A-94/29484
- WO-A-96/01836
- WO-A-96/04404
- WO-A-97/06468
- WO-A-97/07243
- WO-A-98/09735
- WO-A-98/14610
- WO-A-98/20019
- US-A- 5 795 714
- LUTHER ET AL.: "SURFACE-PROMOTED REPLICATION AND EXPONENTIAL AMPLIFICATION OF DNA ANALOGUES" NATURE, Bd. 396, 19. November 1998 (1998-11-19), Seiten 245-248, XP002134112

## Beschreibung

Die vorliegende Erfindung betrifft ein Verfahren zum Klonieren und zum Kopieren genetischen Materials an Oberflächen, aber auch das Kopieren von biologischem Material, soweit es sich in ein Ligand-Rezeptor-System im weiteren Sinne einordnen lässt.

Verfahren zur exponentiellen Amplifikation molekularer Matrizen sind beispielsweise durch G. von Kiedrowski et al. (Nature 1998, Vol. 346, 245-248; DE 198 48 403) bekannt. Die Amplifikationszyklen sind dabei gekennzeichnet durch
a) Binden von molekularen Matrizen an die Oberfläche einer Festphase mittels eines reversiblen Linkers an der Matrize;
b) Zugabe von Matrizenfragmenten, wobei eines der Fragmente eine gegebenenfalls geschützte Linkereinheit aufweist;
c) Synthese von Kopien der Matrize;
d) Entfernen von überschüssigen Matrizenfragmenten und Reaktionshilfsstoffen;
e) Ablösen der Kopien von der Matrize; und
f) Besiedlung freier Bindungsstellen an der Festphase durch synthetisierte Matrizenkopien.

Dieses Verfahren stellt somit ein iteratives schrittweises Amplifikationsverfahren dar, das es erlaubt, die vorhandene Menge an molekularer Matrize exponentiell zu erhöhen und somit einen sinnvollen Evolutionsprozeß ermöglicht. Das Verfahren verwendet dazu die Oberfläche eines festen Trägers. Durch chemische Verknüpfung auf immobilisierten Matrizen werden Kopien aus Vorstufenmatrizen synthetisiert, die dann freigesetzt werden, so dass sie zu neuen Matrizen werden. Der Vorgang kann beliebig oft wiederholt werden.

Weiter ist aus der US 5,641,658 die sogenannte "Bridge"-Technologie bekannt. Hierbei handelt es sich um ein Amplifikationsschema, welches von konventionellen PCR-Methoden ausgeht, sich jedoch zur Aufgabe gesetzt hat, ortsgebundene Amplifikation zu erreichen. Die "Bridge"-Technologie findet vielerlei Verwendung, insbesondere bei analytischen Methoden, die auch mit gewöhnlicher PCR durchgeführt werden könnten, jedoch Separations- und Detektionsschritte der amplifizierten Produkte erleichtert. Diese Technologie zeichnet sich dadurch aus, daß Amplifikation, Selektion und Detektion in einem einzigen Verfahren vereint sind. Weitergehender Stand der Technik ist auf der Homepage der die "Bridge"-Technologie vermarktenden Firma MOSAIC Technologies, Inc. (USA) enthalten (www.mostek.com). Im wesentlichen handelt es sich bei dieser Technologie um folgendes:

Die Bridge-Technologie beschreibt ein Verfahren zur Amplifikation von Nukleinsäuren an Festphasen, wobei beide Amplifikationsprimer kovalent über ihr 5'-Ende an einer einzelnen Festphase gebunden sind. Es handelt sich daher um eine Weiterentwicklung der bekannten Polymerase-Kettenreaktion ("polymerase chain reaction", kurz PCR). Statt in Lösung wird an einer festen Phase PCR durchgeführt. Besonderer Vorteil dieses Verfahrens ist die Fähigkeit, von einer einzelnen Probe eine Vielzahl von verschiedenen genetischen Elementen simultan zu amplifizieren und zu analysieren. Anwendungsgebiete der Bridge-Technologie sind Genexpression, Genomforschung, klinische Diagnostik und Untersuchungen biologischer Flüssigkeiten, wie z.B. Blut. Eine erhöhte Amplifizierungsrate wird durch Eliminierung uneffektiver Primer-Artefakte (wie Primer-Dimere) erreicht Dadurch können einfache, sensitive und kostengünstige DNA-Detektionsmethoden entwickelt werden, z.B. mittels Fluoreszenz. Da die Bridge-Technologie vorsieht, daß sämtliche Amplifikationsprodukte an der festen Phase gebunden bleiben, sind Prolongationsverschmutzungen gering, was wiederum den diagnostischen Wert des Verfahrens im Gegensatz zur herkömmlichen PCR erhöht.

Während das oben beschriebene Verfahren von v. Kiedrowski et al. den Vorteil der Festphasenamplifizierung ganzer Populationen aufweist, weißt das Verfahren gemaß der US 5,641,658 den Vorteil auf, eine einzige Matrize an einer Festphase zu amplifizieren. Nachteilig an diesem Bridge-Verfahren ist, daß eine Amplifizierung mit dem Problem der Produktinhibition verbunden ist, d.h. eine neu hergestellte Kopie kann nicht nur mit dem benachbarten immobilisierten Primer, sondern auch mit dem ebenfalls benachbarten, ursprünglichen Matrizenstrang erfolgen. Weiterer Nachteil ist die untere Längenbegrenzung, die notwendig ist um die Brücke ("Bridging") als Doppelstrang zu erreichen. Femer erfolgt keine Strangtrennung, so daß für den diagnostischen Einsatz die Hybridisierungssignale durch die Hybridisierung mit den komplementären Strängen abgeschwächt wird.

In der WO 94/29484 wird ein Verfahren zur Amplifikation einer Nukleinsäure beschrieben, bei dem der eine Primer an einer Festphase gebunden ist, und der zweite Primer an ein Teilchen gebunden ist, das auf ein magnetisches Feld anspricht. Diese Primer werden in Ziel-Nukleinsäuresequenzen eingebaut. Nach einem Extensionsschritt erfolgt eine Trennung der Nukleinsäurestränge durch Anlegen eines elektromagnetischen Feldes. Der magnetische Primer kann teilchengebunden, somit als eine Art von Festphase vorliegen. Zur Bindung der Primer an den Festphasen ist das Avidin/Biotin-System geeignet. Das Verfahren ist auch zur Klonierung geeignet.

In der US 5,795,714 ist ein Verfahren beschrieben, welches in einer Ausführung ein Array von Oligonukleotiden verwendet, die über Biotin-Avidin-Wechselwirkungen mit der Festphasen-Oberfläche verknüpft sind. Das Verfahren beschreibt in dieser Ausführungsform die Anhybridisierung komplementärer Stränge, Primerextensionsreaktionen, das Anybridiseren eines zweiten biotinylierten Primers an die Primerextensionsprodukte und die Extension des zweiten Primers. Ein blotting der Kopien auf ein zweite mit Avidin beschichtete Oberfläche wird erwähnt. Als nächstliegender Stand der Technik wird daher die US 5,795,714 angesehen.

In der WO 98/01836 wird zwar ein Verfahren zum Kopieren von einer Oberfläche auf eine andere beschreiben. Es wird aber lediglich das Kopieren von einer strukturierten Oberfläche auf eine andere Oberfläche beschrieben, die als "Einmal-Reaktion" durchzuführen ist. Klonierungsreaktionen und ein zyklisiertes Verfahren sind aufgrund der WO 96/01836 nicht möglich.

Eine mehrfache Replikation im Sinne einer exponentiellen Amplifikation ist durch diese Verfahren nicht möglich, genauso wenig wie ein Erhalt der Qrtsinformation bei Translokation der Kopien durch ein elektromagnetisches Feld.

Ausgehend von diesem Stand der Technik ist es daher Aufgabe der vorliegenden Erfindung, ein verbessertes Verfahren zum Kopieren von Nukleinsäuren von einer ersten auf eine zweite Oberfläche zur Verfügung zu stellen, wobei die Ortsinformation erhalten bleibt.

Überraschenderweise wurde ein Verfahren gefunden, welches es erlaubt, Nukleinsäuren, Liganden und Rezeptoren, sowie vergleichbare biologische Systeme zu vermehren und mit Hilfe eines elektrischen Feldes räumlich voneinander zu trennen, sowie auf einer oder mehreren festen Oberflächen nach der Vermehrung unter Erhalt der Ortsinformation zu immobilisieren und damit zu fixieren.

Im Sinne der Erfindung wird unter einem biologischen System grundsätzlich eine Wechselwirkung von Nukleinsäuren jedweder Art untereinander und/oder mit Peptiden/Proteinen/Polymerasen/Enzymen verstanden (DNA/RNA/PNA/pRNA/2' - 5' Nukleotide und RNA/DNA-Spiegelmere (vgl. WO 98/08856)), genauso wie Antigen/Antikörper-Komplexe oder generell Ligand/Rezeptor-Systeme.

Die Erfindung macht sich daher die Tatsache zunutze, dass Nukleinsäuren und zahlreiche andere biologisch relevante Moleküle aufgrund ihrer Ladung durch Anlegen eines elektrischen Feldes in diesem bewegt werden können. Vorliegend werden auf diese Weise ein jeweils stationär gebundenes Molekül von einem korrespondierendem Molekül getrennt, indem das nicht-stationär gebundene Molekül entweder nach Synthetisierung desselben oder nach einer "Identifizierungsreaktion" mit Hilfe des elektrischen Feldes voneinander getrennt werden. Da die Moleküle entlang der Feldlinie des elektrischen Feldes wandern, führt dies zu einer Wanderung der Moleküle unter Erhalt der Ortsinformation. Dies unterscheidet das erfindungsgemäße Verfahren auch wesentlich von vorbekanntem Stand der Technik, da beispielsweise in der WO 94/29484 die Ortsinformation nicht erhalten bleibt; denn das dort beschriebene elektromagnetische Feld dient nicht der Erhalt der Ortsinformation.

Vorzugsweise (aber nicht zwingend) sind die Oberflächen dabei koplanar zueinander angeordnet.

Zum grundsätzlichen Verständnis der Erfindung gehört, dass komplementäre Nukleinsäuren selbst nichts anderes als ein Spezialfall eines komplementären Ligand/Rezeptor-Systems im klassischen Sinn darstellen.

Die Erfindung betrifft daher ganz allgemein Verfahren zur Vermehrung von Liganden und Rezeptoren auf wenigstens zwei Oberflächen vor, umfassend einen oder mehrere der folgenden Zyklen:
a) Immobilisierung eines Liganden an einer ersten Oberfläche einer Festphase;
b) Zugabe einer Lösung von Rezeptoren und Binden komplementärer Rezeptoren an den Liganden;
c) Übertragung des Rezeptors an eine weitere Oberfläche und dortige Immobilisierung des Rezeptors;
d) Anlagerung eines weiteren Liganden an den immobilisierten Rezeptor;
e) Übertragung des Liganden an eine Oberfläche und dortige Immobilisierung des Liganden,
wobei die Übertragung in Schritt (c) und (e) durch Anlegen eines elektrisches Feldes erfolgt.

Die Rolle der Oberfläche (nachfolgend auch "Träger") im erfindungsgemäßen Verfahren besteht darin, komplementäre Matrizen, die in Lösung stabile Duplexe bilden würden, voneinander getrennt zu halten. Geeignete Trägermaterialien bestehen dabei aus organischem oder anorganischem Material oder aus einem Hybrid dieser Materialien. Organische Trägermaterialien sind Polymere auf Zuckerbasis, vorzugsweise Agarose, Cellulose, sowie geeignete Derivate oder technische Polymere wie Polystyrol, Polyacrylat, Polyacrylnitril, Polyalkene oder Pfropfcopolymer (z.B. PS PEG, PAN-PEG, PAN-PAG usw.), aber auch elektrisch leitende Polymere (z.B. Polyvinylpyrrol). Anorganische Trägermaterialien können z.B. Glas sowie Metalle sein,
wobei insbesondere der Goldoberfläche (Infolge Goldthiolatwechselwirkung) und Halbleiteroberflächen besondere Bedeutung zukommt.

Bindungen können mittels kovalenter oder nicht-kovalenter Bindung erfolgen, wobei unter nicht-kovalenter Bindung sowohl ionische als auch nicht-ionische Bindungssysteme und insbesondere Mitglleder von immunologischen Bindungspaaren wie Avidin/Streptavidin oder Antigen-Antikörper umfasst sind. Die Auswahl geeigneter Bindungslinker erfolgt unter dem Gesichtspunkt, dass diese nicht mit übrigen im System auftretenden Faktoren unerwünschte Wechselwirkungen eingehen. Insbesondere dürfen bei der Anhybridisierung eines Primers keine Wechselwirkungen mit Oberflächenbezirken auftreten, die das Templat enthalten. Dies setzt eine steuerbare und durch äußere Bedingungen beeinflußbare Bindungschemie voraus. Die Fehlimmobilisierung kann auch dadurch verhindert werden, dass an Stelle eines "aktivierbaren Reaktivprimers" (siehe unten) Paare von Primem verwendet werden, deren Extensionsprodukte orthogonal immobilisiert werden können. Orthogonal heißt hier, daß für einen Primer, der an ein Templat hybridisiert wird, keine Bindungsstellen vorhanden sind, hingegen nach der Translokation des Primerextensionsproduktes an die gegenüberliegende Oberfläche Bindungsstellen vorhanden sind. Die Art der Primerzugabe und der Reaktionsführung muss diesem Gesichtspunkt Rechnung tragen. Unter aktivierbaren Reaktivprimem werden im Sinne der Erfindung solche Primer verstanden, die eine reaktive Funktionalität besitzen und deren Reaktivität durch die Wahl geeigneter äußerer Bedingungen beeinflussbar ist.

Die Erfindung betrifft insbesondere aber auch ein Verfahren zur enzymatischen Vermehrung von Nukleinsäuren auf wenigstens zwei Oberflächen, umfassend einen oder mehrere der folgenden Amplifikationszyklen:
a) Immobilisierung eines ersten Primers an einer ersten Oberfläche einer Festphase;
b) Zugabe einer Lösung von Nukleinsäuren und Binden komplementärer Fragmente an den ersten Primer;
c) Verlängerung des ersten Primers an seinem 3'-Ende, korrespondlerend zu dem komplementären Fragment, durch eine Polymerase;
d) Freisetzen der komplementären Fragmente;
e) Anlagerung eines zweiten Primers an das 3'-Ende der verlängerten Nukleinsäure;
f) Verlängerung des zweiten Primers an seinem 3'-Ende durch eine Polymerase;
g) Übertragung des zweiten Primers an eine weitere Oberfläche und Immobilisierung des verlängerten Primers;
h) Anlagerung eines weiteren ersten Primers an das 3'-Ende des zweiten, verlängerten Primers,
wobei die Übertragung in Schritt (g) durch Anlegen eines elektrisches Feldes erfolgt.

Daneben beschreibt die Erfindung aber auch in einer bevorzugten Ausführungsform ein Verfahren zum Kopieren von Nukleinsäuren von einer ersten auf eine zweite Oberfläche, umfassend folgende Verfahrensschritte:
a) Immobilisierung von Nukleinsäuren durch Reaktion mit der Oberfläche eines festen Trägers;
b) Erzeugung von doppelsträngigen Molekülen durch
   ba) Hybridisierung komplementärer Einzelstränge; oder
   bb) chemische oder enzymatische Ligation von komplementären Fragmenten; oder
   bc) chemische oder enzymatische Verlängerung von komplementären Primem;
c) Übertragung der Komplementärstränge auf eine zweite Oberfläche und Immobilisierung derselben.
wobei die Übertragung in Schritt (c) durch Anlegen eines elektrisches Feldes erfolgt, wobei das elektrische Feld zwischen der ersten und zweiten Obertläche angelegt wird.

Bei allen vorgenannten erfindungsgemäßen Maßnahmen ist es bevorzugt, die Übertragung mittels eines elektrischen Feldes unter Erhalt der Ortsinformation vorzunehmen. Ebenfalls bevorzugt ist die räumliche Trennung der Oberflächen.

Dieses Verfahren ist nützlich für die Vermehrung von biologischem Material auf sogenannten "Genchips". Dabei ist es grundsätzlich (wie überhaupt in den erfindungsgemäßen Verfahren) unerheblich, ob das 5'-Ende, das 3'-Ende oder eine sequenzinteme Position als Ausgangspunkt für die Verknüpfung mit der Oberfläche dient.

Es ist bereits bekannt, eine große Anzahl immobilislerter polymerer Verbindungen auf einem Objektträger aufzubringen oder zu synthetisieren, um damit selektiv daran bindende Verbindungen nachzuweisen (Fodor et al., Science 251, 767-773, 1991; US 5,510,270, US 5,489,678, US 5,445,934, US 5,424,186). Dazu ist es allerdings zuvor notwendig, für den Herstellungsprozess solcher "Arrays" vori Sonden, lithographische Masken herzustellen und zu verwenden und die monomeren Ausgangsverbindungen mit photolabilen Schutzgruppen zu versehen. Für einen Synthesezyklus bei der Peptidsynthese benötigt man mindestens 20 solcher Masken je Synthesezyklus, bei n Zyklen also n x 20 Masken; bei der Synthese von Oligonukleotiden benötigt man 4 solcher lithographischer Masken, für n Zyklen also n x 4 Masken. Diese lithographischen Masken werden dazu benötigt, um an definierten räumlichen Stellen des "Arrays" eine Belichtung zu erlauben, an allen anderen Stellen des "Arrays" jedoch nicht. An den definierten, belichteten Stellen wird eine lichtempfindliche Schutzgruppe abgespalten und dadurch eine reaktive Gruppe freigesetzt, an die im Anschluss ein neuer monomerer Baustein des Polymers binden kann. Durch vielfaches Anwenden von jeweils individuellen Masken und durch vielfache Wiederholung von Kopplungsprozessen werden solche "Arrays" aufgebaut Derartige Genchips wurden bislang also nach aufwendigen und äußerst kostenintensiven lithographischen Verfahren hergestellt (vgl. hierzu auch die US 5,700,637)).

Die Erfindung bietet daher überraschenderweise eine einfache Alternative zu derartigen Verfahren, die darüber hinaus noch wesentlich effizienter und genauer ist. Aufgrund der Vielzahl von Informationen, die auf derartigen Genchips enthalten sein können, ist es sogar möglich, ganze Gen-Datenbanken, resp. Bibliotheken für Screeningzwecke bereitzustellen. Erfindungsgemäß ist vorgesehen, daß die Nukleinsäuren auf dem festen Träger zweidimensional geordnet angeordnet sind und entsprechend dieser Ordnung übertragen werden. Die zweidimensionale Ordnung kann im Sinne der Erfindung auch als "Unordnung" verstanden werden. Insbesondere bei großen Bibliotheken herrscht zwangsläufig keine Zuordnung hinsichtlich der Bindung einzelner Moleküle an bestimmte Orte. Bei einer Übertragung jedoch werden räumlich gesehen die nicht-zugeordneten Moleküle der Bibliothek mit ihrer ursprünglichen Ortsinformation übertragen. Es handelt sich also um eine Unordnung, die gleichwohl geordnet unter Erhalt der Ortsinformation übertragen wird.

Einsatzgebiete der Erfindung sind beispielsweise die Herstellung von Genchips für diagnostische Zwecke in der Human- und Veterinärmedzin.

In dem erfindungsgemäßen Verfahren können die erzeugten Kopien der Nukleinsäuren identisch oder komplementär zu der Ausgangssequenz (Matrize) sein. Unter "komplementär" im Sinne der Erfindung ist zu verstehen, dass die Kopie der Matrize sich von der Ausgangsmatrize unterscheidet, die Kopie dieser Kopie jedoch wieder identisch mit der Ausgangsmatrize ist Soweit erforderlich wird dies nachfolgend kurz mit "(+)-Strang" und "(-)-Strang" bezeichnet. Die Reaktionen erfolgen im selben Reaktionsgefäß. Unter Nukleinsäuren werden sowohl D- als auch L-Nukleinsäuren (Spiegelmere) verstanden, sowie jedwede Modifikation davon.

Auch können Immunreaktionen in Form von Immunoassays oder Radioimmunoassays (Antikörper-Antigen-Reaktionen) mit diesem erfindungsgemäßem Verfahren durchgeführt werden.

Die Erfindung bietet bislang ungeahnte Vorteile bei allen bekannten - konventionellen - medizinisch-diagnostischen und biochemisch/ biotechnologisch/ gentechnologisch relevanten Methoden, weswegen sich vielfache Anwendungsgebiete anbieten. Beispielhaft seien hier nur folgende Anwendungen genannt.

Das erfindungsgemäße Verfahren kann zur qualitativen und quantitativen Erfassung von DNA und RNA-Molekülen verwendet werden. Mit dem Verfahren können auch komplexe genetische Polymorphismen und multiple Allele simultan analysiert werden. Durch die Vermehrung der DNA oder RNA-Moleküle an festen Phasen können Primerartefakte (z.B. Primer-Dimere) vermieden werden. Ein weiterer Vorteil ist darin zu sehen, dass die zu quantifizierende Probe nur zu Beginn des Verfahrens eingesetzt wird und dann alle Vermehrungsprodukte durch spezifische Bindungen auf den Oberflächen fest verankert sind. Damit können die in der PCR-Diagnostik häufig störenden Signale durch Verunreinigungen vermieden werden. Die Reinheit der Oberflächen kann durch elektrostatische Abstoßung unspezifisch gebundener DNA- oder RNA-Moleküle verbessert werden. Das Verfahren kann daher in der funktionellen Genomik und der Pharmakogenomik eingesetzt werden (vgl. Oliver et al. Trans Biotechnol. 16, 373-378 (1998); Housman and Ledley, Nature Biotechnology 16,492-493 (1998)).

Das erfindungsgemäße Verfahren kann zur Erfassung von differentieller Genexpression genutzt werden. Weiterhin lässt sich das Verfahren mit den im Stand der Technik bekannten Methoden, wie z.B. Differential Display RT-PCR (DDRT-PCR), Serial Analysis of Gene Expression (SAGE) oder differentieller Hybridisierung kombinieren (Wan et al., Nature Biotechnology 14. 1685-1691 (1996)). Durch Vergleich der Genexpression lassen sich beispielsweise neue pharmakologische Wirkorte auffinden.

Zur schnellen qualitativen und quantitativen Erfassung kann das Verfahren mit den im Stand der Technik bekannten Sensorverfahren, wie z.B. Oberflächen-Plasmon-Resonanz-Sensoren, evaneszenten Feld-Sensoren, faseroptische Sensoren, Gitterkopplem oder RIFS (Reflektorische Interferometrische Spektroskopie) kombiniert werden (Scheller et al., Frontiers in Biosensorics, Birkhäuser Verlag Basel (1997)).

Die Erstellung von Gen- und Genombibliotheken nach dem erfindungsgemäßen Verfahren kann mit den im Stand der Technik bekannten Verfahren zur Ligation von Adaptern oder Linkem kombiniert werden. Ein besonderer Vorteil des Verfahrens besteht darin, dass bei Verwendung von zwei verschiedenen Primem für die Immobilisierung an den Oberflächen ausschließlich Moleküle mit zwei verschiedenen A-daptem oder Linkem vermehrt werden. Somit lassen sich die jeweils komplementären Einzelstränge auf den Oberflächen getrennt vermehren.

Darüber hinaus ist auch eine Kombination mit Verfahren möglich, die eine ortsgerichtete lmmobilisierung sowie ein Umsortieren der an einer Oberfläche gebundenen Moleküle gestatten. Hierzu gehören einerseits Methoden, die auf Elektrodenarrays, bei denen eine oder mehrere bestimmte Mikroelektroden gezielt ansteuerbar sind, aufbauen. Die Elektrodenarrays können dabei z.B. durch Halbleiterchips realisiert werden, wie sie von der Firma Nanogen (www.nanogen.com) entwickelt wurden. Andererseits gehören hierzu Methoden, die eine Verwendung von Scanning-Techniken implizieren, bei denen piezoelektrische Elemente für eine höchstpräzise laterale Adressierung bis hin in den Sub-Nanometerbereich sorgen. Bevorzugt ist die Anwendung der Scanning Electrochemical Microscopy (SECM), die zur elektrochemischen Deposition der Moleküle und darüber hinaus in Verbindung mit elektrochemischen Probes zur Detektion einsetzbar ist. Aber auch Methoden wie die Atomic Force Microscopy (AFM) sind geeignet, einzelne Moleküle lateral zu translozieren.

Die nach dem Verfahren erstellten Bibliotheken können mit den im Stand der Technik bekannten Verfahren sequenziert werden, wie z.B. Sequenzlerung durch Didesoxytermination nach Saenger, Sequenzierung durch chemische Spaltung nach Maxam und Gilben, Sequenzierung durch Hybridisierung, Sequenzierung durch Kappilarelektrophorese oder MALDI-Massenspektrometrie (vgl. Adams, Fields, Venter in: Automated DNA Sequencing and Analysis, Academic Press, 1994).

Die erstellten Gen- und Genombibliotheken können für die Zuordnung von DNA- bzw. RNA-bindenden Faktoren eingesetzt werden. So lassen sich z.B. die spezifischen Bindungsorte für Transkriptionsaktivatoren oder Repressoren simultan erfassen. Die verwendeten Gen und Genombibliotheken können dabei einzelsträngig oder doppelsträngig sein.

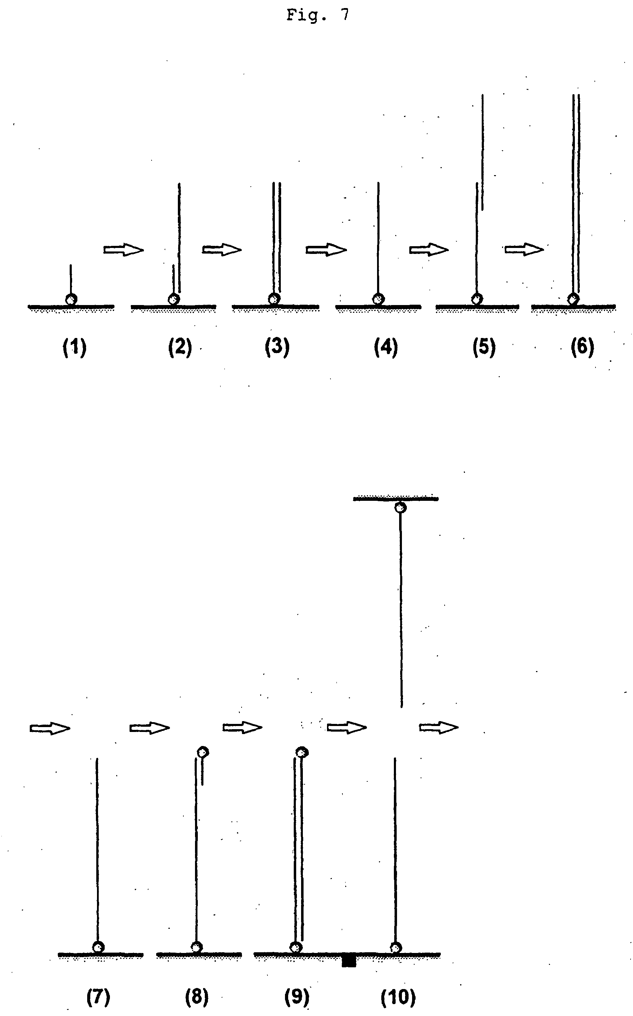

Das Verfahren kann zur Gen und Genomsynthese sowie zur Rekombination genetischen Materials verwendet werden. Die in Fig. 7 (siehe unten) beschriebene Methode erlaubt die Verknüpfung beliebiger Fragmente mit nur partieller Komplementarität. Es bietet sich insbesondere an, offene Leseraster mit geeigneten Startsignalen für biologische Expressionssysteme zu versehen. Weiterhin kann das Verfahren mit der in vitro-Trankription und der in vitro-Translation gekoppelt werden. Da mit dem erfindungsgemäßen Verfahren die Trankriptions- und Translationsprodukte unter Erhalt der Ortsinformation erzeugt werden können, lassen sich räumliche Anordnungen dieser Produkte auf Wechselwirkung mit weiteren Faktoren testen. Damit können funktionelle Zuordnungen simultan erfasst werden, die im Stand der Technik nur durch aufwendige Verfahren, wie z.B. das "Two Hybrid-System" gefunden werden (Fredericson, Curr. Opin. Biotechnol. 9, 90-96 (1998)). Damit können nach dem erfindungsgemäßen Verfahren neue pharmakologische Wirkorte gefunden bzw. neue diagnostische Strategien entwickelt werden.

Das Verfahren kann femer mit dem im Stand der Technik bekannten Methoden zum Auffinden funktioneller Moleküle aus kombinatorischen Bibliotheken kombiniert werden. So kann z.B. ein Zielmolekül (Target) an einer ersten Oberfläche immobilisiert werden. Nach Kontakt mit einer kombinatorischen Nukleinsäure-Bibliothek können die nicht-bindenden RNA- oder DNA-Moleküle abgetrennt werden. Die bindenden Moleküle aus der kombinatorischen Bibliothek werden nach dem erfindungsgemäßen Verfahren auf eine zweite Oberfläche übertragen und unter Erhalt der Ortsinformation vermehrt. Die Zusammensetzung der bindenden Moleküle kann durch Sequenzierung ermittelt werden.

Weiterhin kann durch zyklische Vorgehensweise eine evolutive Optimierung der bindenden Moleküle erfolgen. Die bereits selektierten Moleküle werden erneut mit den immobilisierten Zielmolekülen in Kontakt gebracht und wiederum vermehrt Bei fehlerhafter Vermehrung entstehen Tochtermoleküle mit zum Teil verbesserten Bindungseigenschaften. Die Population der aus der Stammsequenz durch Mutation hervorgehenden Sequenz wird nach Eigen als Quasisequenz bezeichnet. Das erfindungsgemäße Verfahren zeichnet sich nun dadurch aus, daß die Mitglieder der Quasispezies räumlich co-lokalisiert sind, wodurch das Konzept der Quasispezies mit einer lateralen Dimension versehen sind.

Unter immer stringenteren Bedingungen, wie z.B. Abnahme der Target-Konzentration und zunehmendes Spülen, läßt sich eine systematische Optimierung der funktionellen Eigenschaften erreichen. Bei dem erfindungsgemäßen Verfahren kann der Selektionsdruck auch durch zunehmende elektrostatische Abstoßung erreicht werden. Weiterhin läßt sich das Verfahren mit den im Stand der Technik bekannten Methoden zur Selektion katalytischer Nukleinsäuren kombinieren (Tarosow et al., Nature 389, 54-57 (1997)).

Insbesondere bietet sich demnach die Verwendung des Verfahrens zur Klonierung genomischer Fragmente von DNA, cDNA und RNA an. Dies kann insbesondere unter Verwendung von replikativen Polymerasen, z.B. Polymerase III aus *Escherichia coli* geschehen; der Vorteil besteht in einer Vermeidung von Fehlern beim Replizierungsprozeß. Femer bietet sich die Subklonierung nach Restriktionsverdau an. Die Subklonierung spielt eine Rolle bei der Sequenzierung großer genomische Fragmente, wobei der Vorteil bei Verwendung des erfindungsgemäßen Verfahren darin liegt, daß ein erheblicher Zeitgewinn bei der Sortierung großer Nukleinsäurebibliotheken erreichbar ist, wie beispielsweise durch Subklonierungstechniken wie "shotgun doning" oder das Erzeugen gezielter Deletionsvarianten, z.B. Exonuklease-III-Behandlung (vgl. Adams, Flelds, Venter, in: Automated DNA Sequencing and Analysis, Academic Press, 1994).

Aber auch die Verwendung des Verfahrens zum Kopieren von Genchips bietet ungeahnte Möglichkeiten. So können beispielsweise Genchips mit einer Bibliothek von Virusantigenen als Ausgangsmaterial verwendet werden. Durch Reaktion mit Patientenblut und Bindung darin enthaltener Antikörper lässt sich eine Immunreaktion detektieren. Hier greift das erfindungsgemäße Verfahren nach Anspruch 3 ein und führt dazu, dass die identifizierten Antikörper auf eine Oberfläche transferiert werden (via die elektrische Ladung des Antikörpers). Das Verfahren lässt sich beliebig oft wiederholen, was wiederum zu einer Signalverstärkung führt. Somit findet quasi eine lineare Vermehrung statt, die auch schwache Immunsignale identifizierbar macht. Dies macht beispielsweise in der AIDS-Diagnostik Sinn, wo ein besonderes Problem bekanntlich darin liegt, zu Beginn der Infektion dieselbe zu detektieren. Es ist mithin möglich, schon im Frühstadium Krankheiten oder Allergien nachzuweisen. Das erfindungsgemäße Verfahren bietet sich jedoch auch für die exponentielle Vermehrung an, die bei Verwendung eines immobilisierbaren Antigens stattfinden. kann. Ferner kann mittels Halbleitertechnologie und/oder einer Mikromanipulation ("align-Techniken") ein Sortiervorgang auf Genchips vorgenommen werden. Weiterhin besteht die Möglichkeit, die geladenen Nukleinsäuren mit einer positiv geladenen Kopfgruppe zu versehen, die in der Gesamtladung überwiegt. Dadurch kön nen die Moleküle im elektrischen Feld ausgerichtet werden und erlauben damit eine hohe Beladungsdichte auf den Chips (DNA/RNA).

Weitere vorteilhafte Maßnahmen sind in den übrigen Unteransprüchen enthalten. Die Erfindung ist in den anliegenden Zeichnungen dargestellt und wird nachfolgend näher beschrieben:

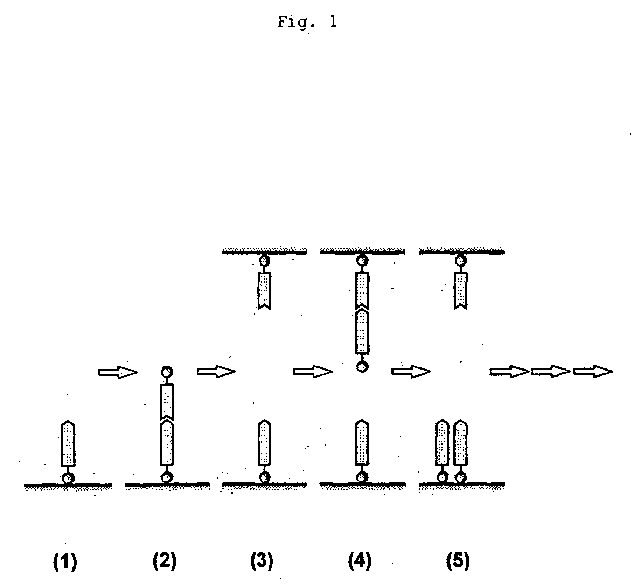

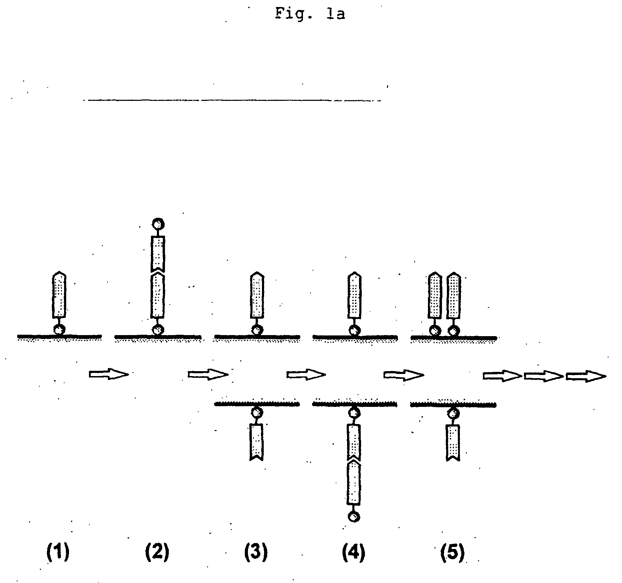

Die Figur 1 beschreibt ein allgemeines Verfahren zur Vermehrung von Liganden und Rezeptoren auf zwei Oberflächen; im einzelnen:
(1) Ein Ugand wird durch eine Reaktion an der Oberfläche eines festen Trägers immobilisiert;
(2) Der Ligand bindet einen Rezeptor;
(3) Der Rezeptor wird auf eine zweite Oberfläche übertragen; dies geschieht wiederum durch Anlegen eines elektrischen Feldes; nach Kontakt mit der zweiten Oberfläche wird der Rezeptor durch eine Reaktion immobilisiert;
(4) Ein freier Ligand wird an den immobilisierten Rezeptor angelagert;
(5) Der Ligand wird auf die zweite Oberfläche übertragen (mit Hilfe eines elektrischen Feldes); dort wird er immobilisiert.

Das Verfahren kann beliebig oft durchgeführt, resp. wiederholt werden.

Die Figur 1a beschreibt das Verfahren gemäß der Figur 1, wie es entsprechend der Figur 3a zu Figur 3 unten beschrieben ist.

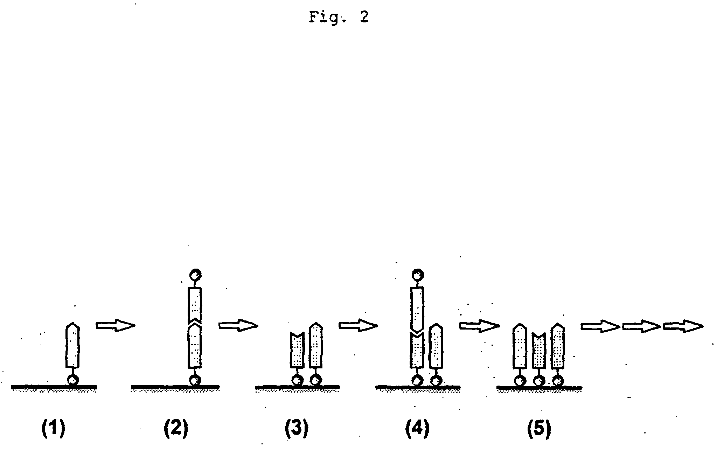

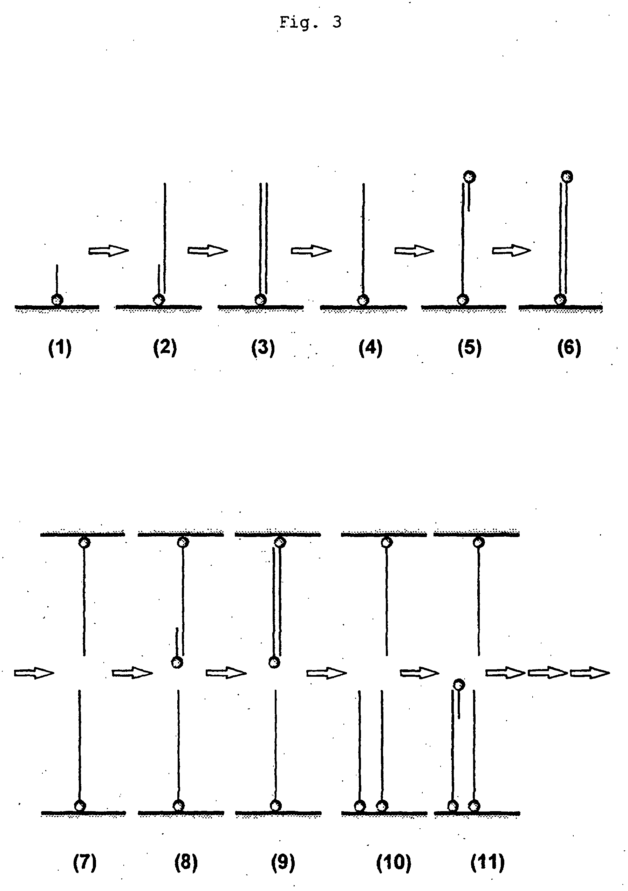

Die Figur 2 beschreibt ein allgemeines Verfahren zur Vermehrung von Liganden und Rezeptoren auf wenigstens zwei Oberflächen; im einzelnen:
(1) Ein Ugand wird durch eine Reaktion an der Oberfläche eines festen Trägers immobilisiert;
(2) Der Ligand bindet einen Rezeptor;
(3) Der Rezeptor wird auf die Oberfläche Obertragen; dies geschieht wiederum durch Anlegen eines elektrischen Feldes; nach Kontakt mit der Oberfläche wird der Rezeptor durch eine Reaktion immobilisiert;
(4) Ein freier Ligand wird an den immobilisierten Rezeptor angelagert;
(5) Der Ligand wird auf der Oberfläche durch eine Reaktion immobilisiert.

Das Verfahren kann beliebig oft wiederholt werden.

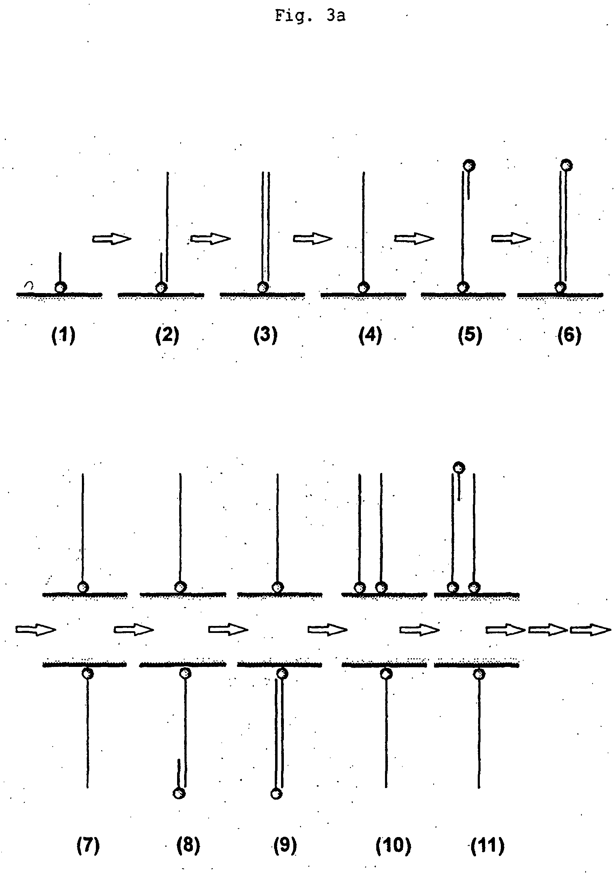

Die Figur 3 beschreibt ein Verfahren zur enzymatischen Vermehrung von Nukleinsäuren auf zwei Oberflächen, wobei die einzelnen Verfahrensschritte als Zeitraffer wie folgt dargestellt sind:
(1) Ein erster Primer A wird durch eine Reaktion an der Oberfläche eines festen Trägers immobilisiert;
(2) Der Primer A bindet aus einer Lösung von Nukleinsäuren komplementäre Fragmente;
(3) Der Primer A wird an seinem 3'-Ende durch eine Polymerase verlängert;
(4) Die komplementären Fragmente werden freigesetzt;
(5) Ein zweiter Primer B wird an das 3'-Ende der verlängerten Nukleinsäure angelagert;
(6) Der Primer B wird an seinem 3'-Ende durch eine Polymerase verlängert;
(7) Der verlängerte, nicht Immobilisierte Primer B wird auf eine zweite Oberfläche übertragen; dies geschieht vorzugsweise durch Anlegen eines etektrischen Feldes, wobei die beiden Oberflächen jeweils entgegengesetzt gepolt sind. Nach Kontakt mit der zweiten Oberfläche wird der verlängerte Primer B durch eine Reaktion dort immobilisiert;
(8) Ein weiterer Primer A wird an das 3'-Ende des verlängerten Primers B angelagert;
(9) Der Primer A wird an seinem 3'-Ende durch eine Polymerase verlängert;
(10) Der verlängerte Primer A wird auf die erste oder eine weitere Oberfläche übertragen. Auch dies geschieht wieder durch Anlegen eines elektrischen Feldes, wobei die beiden Oberflächen jeweils entgegengesetzt gepolt sind, diesmal aber (anders als in Schritt (7)) mit umgekehrten Vorzeichen. Nach Kontakt mit der Oberfläche wird der verlängerte Primer A durch eine Reaktion dort immobilisiert;
(11) Ein weiterer Primer B wird an das 3'-Ende des verlängerten Primers A angelagert.

Das Verfahren wird dann beliebig oft fortgesetzt, vorzugsweise durch zyklisiertes Umpolen.

Die Figur 3a beschreibt das Verfahren gemäß der Figur 3, wobei allerdings zwischen den beiden Oberflächen eine Zwischenschicht angeordnet ist. Diese Zwischenschicht Ist ausgewählt aus der Gruppe eines Gels, einer Membran, eines Polymeren, einer Keramik und/oder eines sogenannten Capillar Tube Arrays. Durch die Zwischenschicht wandert bei Anlegen eines elektrischen Feldes das nicht immobilisierte Molekül auf die zweite Oberfläche, wo es immobilisiert wird.

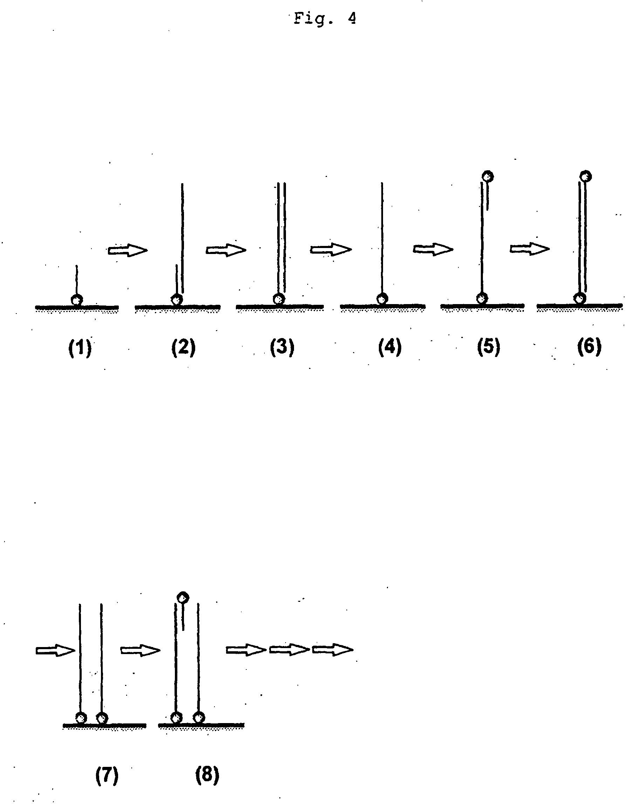

Die Figur 4 beschreibt dagegen zur Veranschaulichung ein grundsätzliches Verfahren zur enzymatischen Vermehrung von Nukleinsäuren auf nur einer Oberfläche; im einzelnen:
(1) Ein erster Primer A wird durch eine Reaktion an der Oberfläche eines festen Trägers immobilisiert;
(2) Der Primer A bindet aus einer Lösung von Nukleinsäuren komplementäre Fragmente;
(3) Der Primer A wird an seinem 3'-Ende durch eine Polymerase verlängert;
(4) Die komplementären Fragmente werden freigesetzt;
(5) Ein zweiter Primer B wird an das 3'-Ende der verlängerten Nukleinsäure angelagert;
(6) Der Primer B wird an seinem 3'-Ende durch eine Polymerase verlängert;
(7) Der verlängerte, nicht immobilisierte Primer B wird an der Oberfläche durch eine irreversible Reaktion gebunden; dies geschieht vorzugsweise durch Anlegen eines elektrischen Feldes, wobei der geladene Primer B entlang des Feldes geführt wird;
(8) Ein weiterer Primer A wird an das 3'-Ende des verlängerten Primers B angelagert;

Das Verfahren kann beliebig fortgesetzt werden.

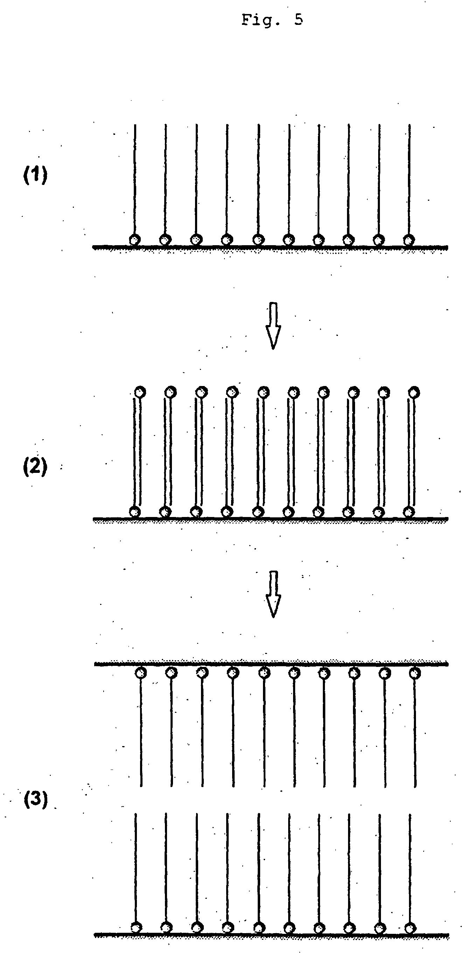

Die Figur 5 hingegen zeigt ein Verfahren zum Kopieren von Nukleinsäuren auf eine zweite Oberfläche; im einzelnen:
(1) Immobilisierung von Nukleinsäuren durch Reaktion mit der Oberfläche eines festen Trägers;
(2) Erzeugung von doppelsträngigen Molekülen durch Hybridisierung komplementärer Einzelstränge; alternativ kann aber auch chemische oder enzymatische Ugation von komplementären Fragmenten erfolgen oder chemische oder enzymatische Verlängerung von komplementären Primem;
(3) Übertragung der Komplementärstränge auf eine zweite Oberfläche und Immobilisierung derselben; dies geschieht vorzugsweise durch Anlegen eines elektrischen Feldes, wobei die beiden Oberflächen jeweils entgegengesetzt gepolt sind.

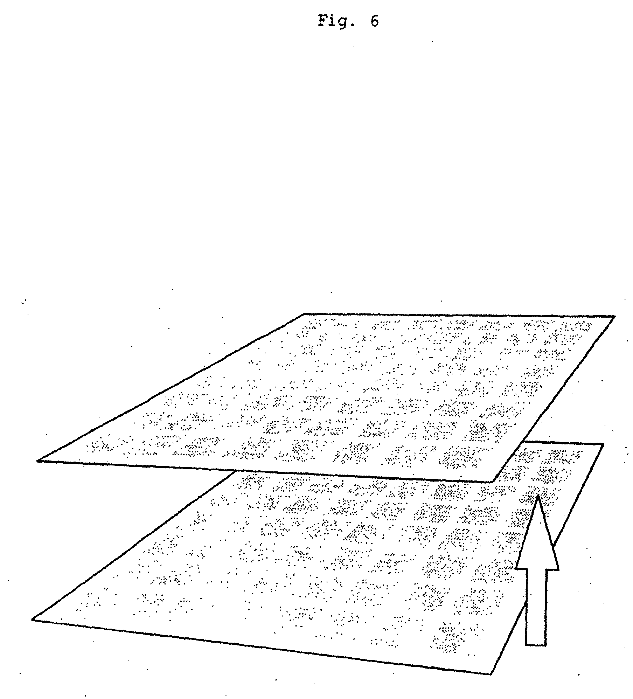

Die Figur 6 zeigt in schematischer Ansicht zwei Koordinatensysteme mit zahlreichen Feldern, die hier zwei Oberflächen versinnbildlichen sollen, wie sie beispielsweise bei Genchips oder auf Membranen zur Anwendung kommen. Die untere Oberfläche enthält die zu kopierende Information, die durch Anlegen eines (nicht dargestellten) elektrischen Feldes entsprechend dem Verfahren nach Figur 3 auf die obere Oberfläche übertragen und dort immobilisiert wird.

Die Figur 7 zeigt ein Verfahren zur Gen- und Genom-Synthese, sowie zur Rekombination; im einzelnen:
(1) Ein Primer A wird durch eine Reaktion an der Oberfläche eines festen Trägers immobilisiert;
(2) Der Primer A bindet aus einer Lösung von Nukleinsäuren komplementäre Fragmente;
(3) Der Primer A wird an seinem 3'-Ende durch eine Polymerase verlängert;
(4) Die komplementären Fragmente werden freigesetzt.
(5) Ein zweites Fragment, das zum 3'-Ende des verlängerten Primers A komplementär ist, wird angelagert, wobei auch teilkomplementäre Fragmente ausreichen, deren 3'-Ende überhängen;.
(6) Der verlängerte Primer A wird elongiert.
(7) Die komplementären Fragmente werden freigesetzt. Die Schritte (5)-(7) können beliebig oft wiederholt werden.
(8) Ein zweiter Primer B wird an das 3'-Ende der verlängerten Nukleinsäure angelagert.
(9) Der Primer B wird an seinem 3-Ende durch eine Polymerase verlängert.
(10) Der verlängerte Primer B wird wie in Fig. 1, Schritt 7 beschrieben auf eine zweite Oberfläche übertragen. Dieser Schritt hat den Vorteil, daß unvollständig verlängerte Primer A-Moleküle abgetrennt werden. Der verlängerte Primer B kann erneut für Verlängerungsreaktionen eingesetzt werden.

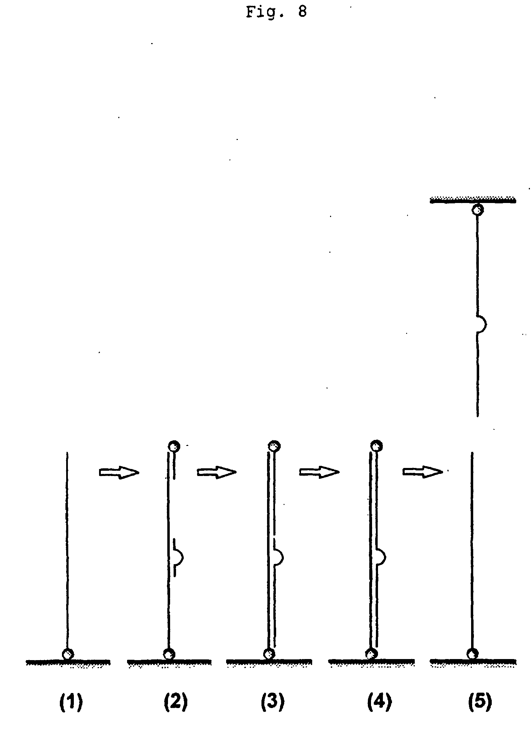

Die Figur 8 zeigt ein Verfahren zur gezielten Mutagenese von Nukleinsäuren (sitedirected Mutagenesis); im einzelnen:
(1) Eine Nukleinsäure A wird durch eine Reaktion an der Oberfläche eines festen Trägers immobilisiert; die Nukleinsäure kann eines der Produkte der vorgenannten Verfahren sein;
(2) Die Nukleinsäure A bindet aus einer Lösung von Nukleinsäuren komplementäre Fragmente, die Fehlbasenpaarungen aufweisen (Mutationsfragment); weiterhin wird ein Primer B angelagert; das Mutationsfragment kann auch identisch mit dem Primer B sein;
(3) Das Mutationsfragment und der Primer B werden verlängert;
(4) Die verlängerten Moleküle werden miteinander ligiert;
(5) Der verlängerte Primer B wird wie in Fig. 1, Schritt 7 beschrieben auf eine zweite Oberfläche übertragen; diese Vorgehensweise hat den Vorteil, daß auf der zweiten Oberfläche nur mutierte Moleküle vorliegen.

Die Erfindung sei an folgendem Ausführungsbeispiel erläutert, nämlich an einem Verfahren zur Vermehrung von Nukleinsäuren an zwei Oberflächen:

Die Synthese des Primers A führte zu einem Biotin-Label an seinem 5'-Ende; hingegen enthielt der Primer B an seinem 5'-Ende ein Fluoreszein-Label. Die Label wurden nach im Stand der Technik üblichen Verfahren, wie z.B. der Phospoamiditchemie hergestellt. Der Primer A wurde an eine Membran A gekoppelt. Als Membran wurde eine für Nukleinsäuren durchlässige bekannte Papiermembran verwendet, die hier als Oberfläche fungierte.

Diese Membran enthielt kovalent gekoppeltes Streptavidin. Durch die Biotin/Streptavidin-Wechselwirkung fand die Kopplung statt. Ein zum Primer A komplementärer DNA-Strang wurde an diesen Primer A anhybridisert. Der Primer A wurde durch Taq-Polymerase oder durch das Klenow-Fragment der Polymerase I am 3'-Ende verlängert Anschließend wurde durch Erhitzen auf 90°C denaturiert (alternativ kann auch eine Denaturierung durch ein übliches Denaturierungsreagenz erfolgen). Zum Anhybridiseren des Primers B wurde die Denaturierungslösung gegen eine Lösung, enthaltend TRIS-Borat-EDTA-Puffer ausgetauscht. Der Primer B wurde anschließend ebenso verlängert wie zuvor der Primer A (vgl. Figur 3 und 4).

In einer herkömmlichen Elektrophoreseapparatur wurde die Membran A auf ein Gel, vorzugsweise PAGE-Gel, gelegt. Auf der Rückseite des Gels wurde vorher eine Membran B aufgebracht, an der zuvor Fluoreszein-Antikörper immobilisiert wurden. Dieses "Sandwich" wurde mechanisch in einem Rahmen gehalten, stabilisiert und in eine Elektrophoresekammer eingeführt, die dergestalt war, daß die beiden Puffer voneinander getrennt waren, d.h., daß das Sandwich den Anoden- und Kathodenraum trennte. Bei Anlegen einer Spannung, die der Dicke des Gels angepaßt war (hier 300 V), wurde nachfolgend in das Elektrolyt ein denaturierendes Agens in den (-)-Raum eingespült (z.B. Hamstofflösung). Nach Erhitzen auf 70°C wurde e-lektrophoretisiert. Dabei löste sich der verlängerte Primer B und wanderte durch die Gelschicht auf die Membran B, wo er anschließend durch Bindung an den Fluoreszein-Antikörper gebunden und immobilisert wurde.

Anschließend wurde das Sandwich aus der Elektrophoresekammer entfernt, die Membranen vom Gel entfernt und an der Membran B wurde nach den oben beschriebenen Methoden der Primer A wiederum anhybridisert und verlängert. Beide Membranen wurden auf ein frisches PAGE-Gel gelegt, in der Gestalt, daß die belden Membranen in der ursprünglichen Orientierung unter Erhalt der Ortsinformationen, paßgenau ausgerichtet wurden. Anschließend wurde wie oben beschrieben die Elektrophorese durchgeführt, allerdings in der Weise, daß die vorher im (+)-Raum (Anodenraum) befindliche Membran B nunmehr im (-)-Raum (Kathodenraum) angeordnet war.

Alternativ kann das Experiment in einer Mikrofluid-Apparatur durchgeführt werden, bei dem die Membranen und die Elektroden fest orientiert sind, der Anoden- und Kathodenraum jedoch unterschiedlich voneinander gespült und mit den entsprechenden Reagenzien gespült werden kann. In dieser Apparatur verwendet man schwach quervernetzte Gele, wie sie in der Kapillarelektrophorese üblich sind. Diese Gel werden dann bei jedem Vorgang ausgewechselt.

Ferner kann das Experiment alternativ mit aktiverbaren Reaktivprimem durchgeführt werden. Unter aktivierbaren Reaktivprimem werden im Sinne der Erfindung solche Primer verstanden, die eine reaktive Funktionalität besitzen und deren Reaktivität durch die Wahl geeigneter äußerer Bedingungen beeinflußbar ist. Solche äußere Bedingungen können chemischer, elektrochemischer oder photochemischer Art sein. Ein Beispiel für einen aktivierbaren Reaktivprimer ist ein Oligonukleotid, das über einen Aminolinker eine Cysteineinheit besitzt, deren Thiolgruppe als 2'-Thiopyridyldisulfidgruppe geschützt ist. Die Membran enthält in diesem Fall Carboxygruppen in Form reaktiver Thioester. Beim Anhybridisieren werden redoxneutrale Reaktionsbedingungen verwendet. Nach Durchwandern des Feldes gelangt der verlängerte Primer auf eine Membran, an/in der reduzierende Bedingungen herrschen. Reduktive Bedingungen werden geschaffen durch die Vorlage oder Anwesenheit von z.B. Thiolen wie Dithioerytrol oder Dithiotreitol. In Gegenwart dieser Reagenzien kommt es unter Disulfidaustauschreaktionen zu einer Abspaltung von 2-Thiopyridon, so daß die nunmehr freigesetzte Thiolgruppe am verlängerten Primer mit dem Thioester an der Membran reagieren kann. Bei der Reaktion wird primär ein Thioester gebildet, der durch die benachbarte intramolekulare Aminogruppe des Cysteins zum Amid abreagiert.

Weitere Verwendungsmöglichkeiten der Erfindung sind in den folgenden Figuren wie folgt beschrieben:

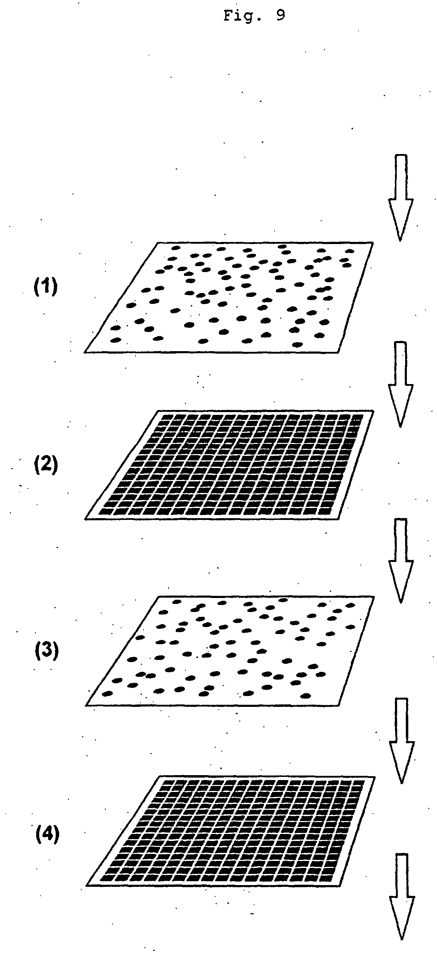

Die Figur 9 zeigt ein Verfahren zur Klonierung und Sequenzierung genomischer Fragmente.
(1) Nach Restriktionsverdau werden die DNA-Fragmente mit zwei verschiedenen Linkem ligiert, die die Sequenz zu zu verwendenden Primer A und B vorgeben. Die genomischen Fragmente werden durch das Verfahren gemäß Fig. 3 vereinzelt. Bei der Vermehrung werden nur die Fragmente amplifiziert, die verschiedene Linker tragen.
(2) Die so vereinzelten und amplifizierten Fragmente werden durch im wesentlichen bekannte Hybridisierungstechniken sortiert ("chromosome walking").
(3) Die sortierten Fragmente werden einzeln vermehrt, mit Restriktionsendonukleasen gespalten und subkloniert.
(4) Die subklonierten Fragmente können emeut durch Hybridisierungstechniken sortiert werden. Die Fragmente werden dann der Sequenzanalyse zugeführt.

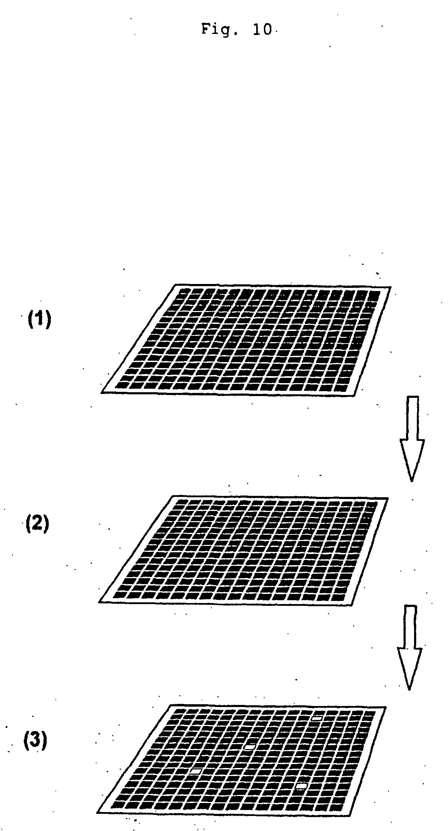

Die Figur 10 zeigt ein Verfahren zur funktionellen Analyse von genomischen Fragmenten.
(1) DNA Fragmente werden, wie in Figur 9 beschrieben, sortiert.
(2) Die einzelsträngigen Fragmente werden durch chemische oder enzymatische Synthese zum Doppelstrang ergänzt.
(3) Die Fragmente werden mit Faktoren (z.B. Repressorprotelne, Aktivatorproteine) in Kontakt gebracht. Der Nachweis der spezifischen Bindung an bestimmte Nukleinsäurefragmente erlaubt die funktionelle Zuordnung des Faktors im genomischen Kontext.

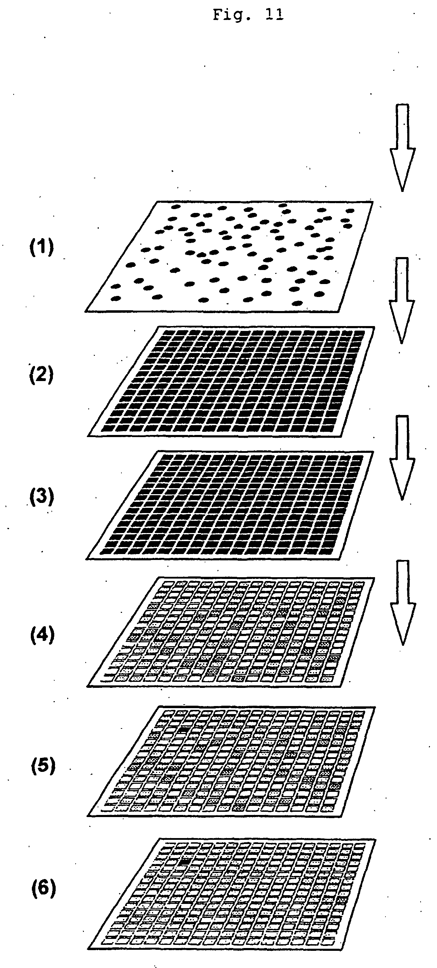

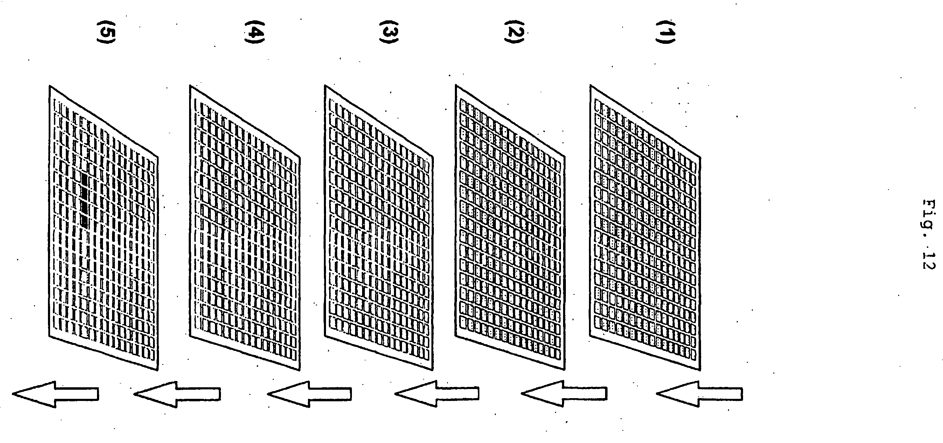

Die Figur 11 zeigt ein Verfahren zur parallelen Quantifizierung der Genexpression.
(1) Nach reverser Transkription von mRNA werden die DNA-Fragmente, wie in Figur 9 beschrieben, mit Linkern versehen und vereinzelt.
(2) Die cDNA-Fragmente werden sortiert.
(3) Die cDNA-Ragmente werden sequerziert.
(4) Kopien der sortierten und sequenzierten Bibliotheken werden mit zellulären mRNAs einer gesunden Zelle in Kontakt gebracht. Die spezifischen Hybridisierungsereignisse werden durch im Stand der Technik bekannte Verfahren (z.B. Fluoreszente Reportergruppen) nachgewiesen.
(5) In analoger Weise werden die zellulären mRNAs einer pathologisch veränderten Zelle (z.B. Tumorzelle) mit einer weiteren Kopie der Bibliothek in Kontakt gebracht.
(6) Der Vergleich der derartig quantifizierten Genexpressionsmuster erlaubt die Identifizierung der mit der Erkrankung verbundenen Gene.

Die Figur 12 zeigt die Verwendung des Verfahrens zur Verbesserung des Signal-Rausch-Verhältnisses beim Nachweis .
(1) Eine Bibliothek wird nach dem in Figur 9 oder Figur 11 beschriebenen Verfahren hergestellt.
(2) Die Bibliothek wird mit der zu analysierenden einzelsträngigen DNA oder RNA in Kontakt gebracht.
(3) Die hybridisierenden DNAs oder RNAs werden auf eine gegenüberliegende Oberfläche unter Erhalt der Ortsinformation übertragen.
(4) Die Schritte 2 und 3 werden wiederholt.
(5) Der Schritt 4 kann beliebig oft durchgeführt, resp. wiederholt werden. Die Signalmessung kann unter Verwendung von sensitiven Scanning-Techniken, wie z.B. Scanning Elecctrochemical Microscopy (SECM) oder Atomic Force Microscopy (AFM) erfolgen.

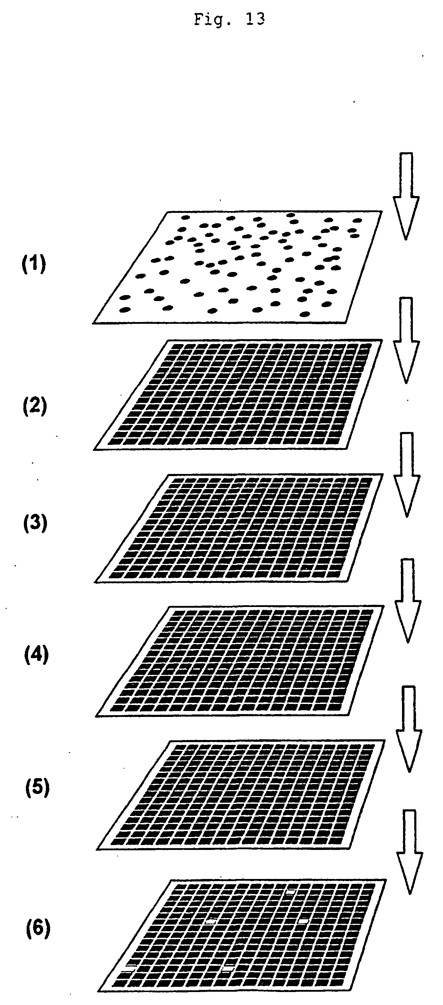

Die Figur 13 zeigt die Verwendung des Verfahrens zur funktionellen Zuordnung von Proteinen.
(1) Eine Bibliothek wird nach dem in Figur 9 oder Figur 11 beschriebenen Verfahren hergestellt, wobei einer der beiden verwendeten Linker eine Startsequenz für eine RNA Polymerase enthält. Der Promotor kann auch nachträglich durch das in Figur 7 beschriebene Verfahren angehängt werden.
(2) Die DNA-Fragmente werden sortiert.
(3) Die einzelsträngigen Fragmente werden durch chemische oder enzymatische Synthese zum Doppelstrang ergänzt.
(4) Die doppelsträngigen DNA-Fragmente werden durch in vitro-Transkription in RNA übersetzt. Die entstehenden RNAs werden durch Anlegen eines elektrischen Feldes unter Erhalt der Ortsinformation auf eine neue Oberfläche übertragen.
(5) Die RNA-Bibliothek wird durch in vitro-Translation in Proteine übersetzt. Die entstehenden Proteine werden durch Anlegen eines elektrischen Feldes unter Erhalt der Ortsinformation auf eine neue Oberfläche übertragen. Da Proteine sehr unterschiedliche Nettoladungen aufweisen können, wird der in vitro Translationsschritt vorzugsweise wiederholt, wobei der Transferschritt mit entgegengesetzter Polarität durchgeführt wird.
(6) Die Proteinbibliothek wird mit einem oder mehren Faktoren (Proteine, RNAs, DNAs, andere Moleküle biologischen oder chemischen Ursprungs) in Kontakt gebracht Die spezifischen Bingungssereignisse werden durch im Stand der Technik bekannte Verfahren nachgewiesen. Der Nachweis der spezifischen Bindung erlaubt die simultane Erfassung funktioneller Wechselwirkungen.

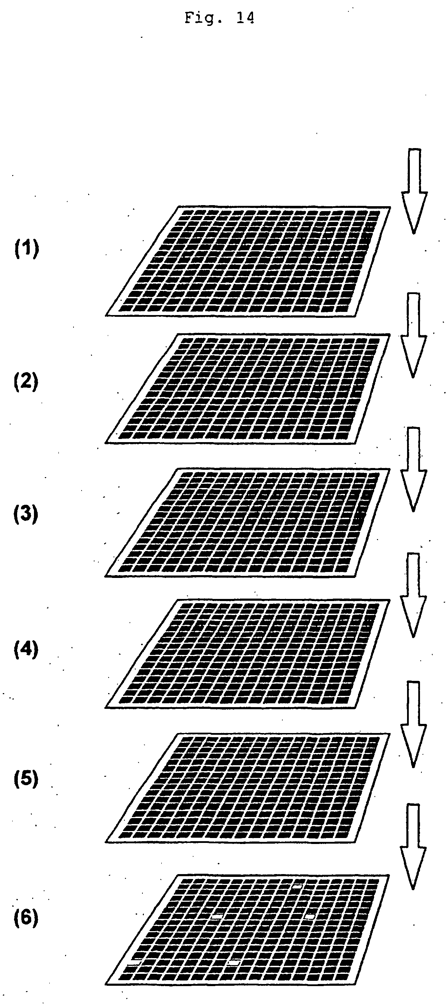

Die Figur 14 zeigt die Verwendung des Verfahrens zum Screening von kombinatorischen Proteinbibliotheken.
(1) Eine Oligonukleotidbibliothek wird nach im Stand der Technik bekannten Verfahren durch chemische Synthese hergestellt.
(2) Die Oligonukleoudbibliothek wird durch das in Figur 7 beschriebene Verfahren in 3'- und 5'-terminaler Richtung verlängert. Die verlängernden Sequenzen kodieren z.B. für die konstanten Regionen eines single chain-Antikörpers.
(3) Die einzelsträngigen Fragmente werden durch chemische oder enzymatische Synthese zum Doppelstrang ergänzt.
(4) Die doppelsträngigen DNA-Fragmente werden durch In vitro-Transkription in RNA übersetzt. Die entstehenden RNAs werden durch Anlegen eines elektrischen Feldes unter Erhalt der Ortsinformation auf eine neue Oberfläche übertragen.
(5) Die RNA-Bibliothek wird durch in vitro-Translation in Proteine übersetzt. Die entstehenden Proteine werden durch Anlegen eines elektrischen Feldes unter Erhalt der Ortsinformation auf eine neue Oberfläche übertragen.
(6) Die Proteinbibliothek wird mit einem oder mehren Faktoren (Proteine, RNAs, DNAs, andere Moleküle biologischen oder chemischen Ursprungs) in Kontakt gebracht. Die spezifischen Bingungssereignisse werden durch im Stand der Technik bekannte Verfahren nachgewiesen. Der Nachweis der spezifischen Bindung erlaubt die simultane Erfassung funktioneller Wechselwirkungen.

Bei allen vorgestellten Verfahrensaltemativen ist es möglich innerhalb des jeweiligen Übertragungsschritts des jeweiligen Verfahrensablaufs den (geometrischen) Maßstab der Übertragung unter Erhalt der Ortsinformation zu verändern, resp. zu verkleinern und/oder zu vergrößern. Dies ist z.B. dann zweckmäßig, wenn die Geometrie der Arrays, von denen übertragen wird bzw. auf die übertragen wird, nicht identisch mit dem Ausgangsarray bzw. Zielarray sind.

## Patentansprüche

1. Verfahren zur Vermehrung von Liganden und Rezeptoren auf wenigstens zwei Oberflächen, umfassend einen oder mehrere der folgenden Zyklen:
a) Immobilisierung eines Liganden an einer ersten Oberfläche einer Festphase;
b) Zugabe einer Lösung von Rezeptoren und Binden komplementärer Rezeptoren an den Liganden;
c) Übertragung des Rezeptors an eine weitere Oberfläche und dortige Immobilisierung des Rezeptors;
d) Anlagerung eines weiteren Liganden an den immobilisierten Rezeptor;
e) Übertragung des Liganden an eine beliebige weitere Oberfläche und dortige Immobilisierung des Liganden,
wobei die Übertragung in Schritt (c) und (e) durch Anlegen eines elektrisches Feldes zwischen der ersten und zweiten Oberfläche erfolgt.

2. Verfahren zur enzymatischen Vermehrung von Nukleinsäuren auf wenigstens zwei Oberflächen, umfassend einen oder mehrere der folgenden Amplifikationszyklen:
a) Immobilisierung eines ersten Primers an einer ersten Oberfläche einer Festphase;
b) Zugabe einer Lösung von Nukleinsäuren und Binden komplementärer Fragmente an den ersten Primer;
c) Verlängerung des ersten Primers an seinem 3'-Ende, korrespondierend zu dem komplementären Fragment, durch eine Polymerase;
d) Freisetzen der komplementären Fragmente;
e) Anlagerung eines zweiten Primers an das 3'-Ende der verlängerten Nukleinsäure;
f) Verlängerung des zweiten Primers an seinem 3'-Ende durch eine Polymerase;
g) Übertragung des zweiten Primers an eine weitere Oberfläche und Immobilisierung des um den zweiten Primer verlängerten ersten Primers.
h) Anlagerung eines weiteren ersten Primers an das 3'-Ende des zweiten, verlängerten Primers;
wobei die Übertragung in Schritt (g) durch Anlegen eines elektrisches Feldes zwischen der ersten und zweiten Oberfläche erfolgt.

3. Verfahren nach Anspruch 2, wobei nach Übertragung auf die zweite Oberfläche die folgenden Amplifikationsschritte erfolgen:
i) Verlängerung dieses ersten Primers an seinem 3'-Ende, korrespondierend zu dem komplementären Fragment, durch eine Polymerase;
j) Übertragung des verlängerten Primers auf die erste oder eine weitere Oberfläche und Immobilisierung des Primers;
k) Anlagerung eines weiteren zweiten Primers an das 3'-Ende des verlängerten ersten Primers.

4. Verfahren zum Kopieren von Nukleinsäuren von einer ersten auf eine zweite Oberfläche, umfassend folgende Verfahrensschritte:
a) Immobilisierung von Nukleinsäuren durch Reaktion mit der Oberfläche eines festen Trägers;
b) Erzeugung von doppelsträngigen Molekülen durch
ba) Hybridisierung komplementärer Einzelstränge; oder
bb) chemische oder enzymatische Ligation von komplementären Fragmenten; oder
bc) chemische oder enzymatische Verlängerung von komplementären Primern;
c) Übertragung der Komplementärstränge auf eine zweite Oberfläche und Immobilisierung derselben,
wobei die Übertragung in Schritt (c) durch Anlegen eines elektrisches Feldes erfolgt, wobei das elektrische Feld zwischen der ersten und zweiten Oberfläche angelegt wird.

5. Verfahren nach Anspruch 4, wobei die Nukleinsäuren auf dem festen Träger zweidimensional geordnet angeordnet sind und entsprechend dieser Ordnung unter Erhalt der Ortsinformation übertragen werden.

6. Verfahren nach wenigstens einem der vorhergehenden Ansprüche, wobei das Festphasenmaterial ausgewählt ist aus organischem oder anorganischem Material oder aus einem Hybrid dieser Materialien und eine zweioder dreidimensionale Matrix darstellt.

7. Verfahren nach wenigstens einem der vorhergehenden Anspruch, wobei die Immobilisierung durch kovalente oder nicht-kovalente Bindung erfolgt.

8. Verfahren nach wenigstens einem der vorhergehenden Ansprüche, wobei die Nukleinsäuren, Liganden, Rezeptoren oder deren Derivate mit einem detektierbaren Label versehen sind.

9. Verfahren nach Anspruch 8, wobei das Label ausgewählt ist aus der Gruppe der Radioisotope, der stabilen Isotope, der Enzyme, der immunreaktiven Verbindungen, der Fluoreszenz- oder Lumineszenz-Chemikalien, der Chromophore, der Metalle oder geladener Teilchen.

10. Verfahren nach wenigstens einem der vorhergehenden Ansprüche, wobei die Lösung von Nukleinsäuren D- und/oder L-Nukleinsäuren umfasst.

11. Verfahren nach wenigstens einem der vorhergehenden Ansprüche, wobei zwischen den Oberflächen eine für die Liganden/Rezeptoren durchlässige Zwischenschicht angeordnet ist.

12. Verfahren nach Anspruch 11, wobei die Zwischenschicht ausgewählt ist aus der Gruppe eines Gels, einer Membran, eines Polymeren, einer Keramik und/oder eines Capillar Tube Arrays.

13. Verfahren nach wenigstens einem der vorhergehenden Ansprüche, wobei die Nukleinsäuren mit einer positiv geladenen Kopfgruppe versehen sind.

14. Verwendung des Verfahrens nach den Ansprüchen 2 oder 3 zur Klonierung genomischer Fragmente von DNA, cDNA und RNA.

15. Verwendung nach Anspruch 14 zur Subklonierung nach Restriktionsverdau.

16. Verwendung des Verfahrens nach Anspruch 1 zur Verstärkung des Ligandensignals.

17. Verwendung des Verfahrens nach Anspruch 4 zum Kopieren von Genchips.

## Claims

1. A method for propagating ligands and receptors on at least two surfaces, encompassing one or more of the following cycles:
a) Immobilization of a ligand on a first surface of a solid phase;
b) Adding a solution of receptors and binding complementary receptors to the ligands;
c) Transferring the receptor to an additional surface and immobilizing the receptor at that location;
d) Attaching an additional ligand to the immobilized receptor;
e) Transferring the ligand to an arbitrary another surface and immobilizing it at that location.
whereby the transfer in stage (c) and (e) is achieved by the application of an electrical field, in which the electrical field is applied between the first and second surface.

2. Method for the enzymatic propagation of nucleic acids on at least two surfaces, encompassing one or more of the following amplification cycles:
a) Immobilization of a first primer on one of the first surfaces of a solid phase;
b) Administration of a solution of nucleic acids and binding of complementary fragments to the first primer;
c) Extension of the first primer at its 3' end, corresponding to the complementary fragment, by means of a polymerase;
d) Release of the complementary fragments;
e) Attaching a second primer to the 3' end of the extended nucleic acid;
f) Extension of the second primer at its 3' end by means of a polymerase;
g) Transfer of the second primer to another surface and immobilization of the first primer, which is extended by the second primer;
h) Attaching another first primer to the 3' end of the second, extended primer;
whereby the transfer in stage (g) is achieved by the application of an electrical field, in which the electrical field is applied between the first and second surface.

3. Method according to Claim 2, in which the following amplification stages occur following the transfer to the second surface:
i) Extension of this first primer to its 3' end, corresponding to the complementary fragment, by means of a polymerase;
j) Transfer of the extended primer to the first or another surface and immobilization of the primer;
k) Attachment of another second primer to the 3' end of the extended first primer.

4. Method for copying nucleic acids from a first to a second surface, encompassing the following stages of the method:
a) Immobilization of nucleic acids through a reaction with the surface of a solid carrier;
b) Production of double-stranded molecules by
ba) hybridization of complementary single strands; or
bb) chemical or enzymatic ligation of complementary fragments; or
bc) chemical or enzymatic extension of complementary primers;
c) Transfer of complementary strands to a second surface, and their immobilization.
in which the transfer in stage (c) occurs by the application of an electrical field, in which the electrical field is applied between the first and second surface.

5. Method according to Claim 4, in which the nucleic acids on the solid carrier are arranged two-dimensionally and are transferred in this order, while retaining site information.

6. Method according to at least one of the previous claims, in which the solid phase material is selected from organic or inorganic material or from a hybrid of these materials, and represents a two- or three-dimensional matrix.

7. Method according to at least one of the previous claims, in which the immobilization takes place through covalent or non-covalent binding.

8. Method according to at least one of the previous claims, in which the nucleic acids, ligands, receptors or their derivatives are provided with a detectable label.

9. Method according to Claim 8, in which the label is selected from the group of radioisotopes, stable isotopes, enzymes, immunoreactive compounds, fluorescence or luminescence chemicals, chromophores, metals or charged particles.

10. Method according to at least one of the previous claims, in which the solution of nucleic acids includes D- and/or L-nucleic acids.

11. Method according to at least one of the previous claims, in which an intermediate layer that can be permeated by nucleic acids and/or ligands/receptors is placed between the surfaces.

12. Method according to Claim 11, in which the intermediate layer is selected from the group comprising a gel, a membrane, a polymer, a ceramic and/or a so-called capillary tube array.

13. Method according to at least one of the previous claims, in which the nucleic acids are provided with a positively charged group of headings.

14. Use of the method according to Claims 2 or 3 for cloning genomic fragments of DNA, cDNA and RNA.

15. Use in accordance with Claim 14 for subcloning following restriction-digesting.

16. Use of the method according to Claim 1 for strengthening the ligand signal.

17. Use of the method according to Claim 4 for the copying of gene chips.

## Revendications

1. Procédé pour la multiplication de ligands et de récepteurs sur au moins deux surfaces, englobant un ou plusieurs des cycles suivants :
a) immobilisation d'un ligand sur une première surface d'une phase solide ;
b) addition d'une solution de récepteurs et fixation de récepteurs complémentaires aux ligands ;
c) transfert du récepteur sur une autre surface et immobilisation sur place du récepteur ;
d) dépôt d'un autre ligand au récepteur immobilisé ;
e) transfert du ligand sur n'importe qu'elle autre surface et immobilisation sur place du ligand,
le transfert au cours des phases (c) et (e) s'effectuant en appliquant un champs électrique entre la première et la deuxième surface.

2. Procédé pour la multiplication enzymatique des acides nucléiques sur au moins deux surfaces, englobant un ou plusieurs des cycles d'amplification suivants :
a) immobilisation d'une première amorce sur une première surface d'une phase solide ;
b) addition d'une solution d'acides nucléiques et fixation de fragments complémentaires à la premières amorce ;
c) élongation par une polymérase de la première amorce à son extrémité 3', correspondant au fragment complémentaire ;
d) dégagement des fragments complémentaires ;
e) dépôt d'une deuxième amorce à l'extrémité 3' de l'acide nucléique élongée ;
f) élongation par une polymérase de la deuxième amorce à son extrémité 3';
g) transfert de la deuxième amorce sur une autre surface et immobilisation de la première amorce élongée de la deuxième amorce ;
h) dépôt d'une autre première amorce à l'extrémité 3' de la deuxième amorce élongée ;
le transfert au cours de la phase (g) étant effectuée en appliquant un champs électrique entre la première et la deuxième surface.

3. Procédé selon la revendication 2, les phases d'amplification suivantes s'effectuant après le transfert sur la deuxième surface :
i) élongation par une polymérase de cette première amorce à son extrémité 3', correspondant au fragment complémentaire ;
j) transfert de l'amorce élongée sur la première surface ou sur une autre surface et immobilisation de l'amorce ;
k) dépôt d'une autre deuxième amorce à l'extrémité 3' de la première amorce élongée.

4. Procédé pour copier des acides nucléiques d'une première surface sur une deuxième surface, englobant les phases de procédé suivantes :
a) immobilisation des acides nucléiques par une réaction avec la surface d'un support solide ;
b) production de molécules à brins doubles par
ba) hybridation de brins simples complémentaires ; ou
bb) ligation chimique ou enzymatique de fragments complémentaires ; ou
bc) élongation chimique ou enzymatique d'amorces complémentaires ;
c) transfert de brins complémentaires sur une deuxième surface et immobilisation des mêmes brins,
le transfert au cours de la phase (c) s'effectuant en appliquant un champs électrique entre la première et la deuxième surface.

5. Procédé selon la revendication 4, les acides nucléiques étant placés sur le support solide, disposés sur deux dimensions et transmis dans cet ordre selon la réception de l'information locale.

6. Procédé selon au moins une des revendications précédentes, le matériau de la phase solide étant choisi parmi un matériau organique ou anorganique ou un hybride de ces matériaux et représentant une matrice à deux ou trois dimensions.

7. Procédé selon au moins une des revendications précédentes, l'immobilisation étant effectuée par une fixation covalente ou non-covalente.

8. Procédé selon au moins une des revendications précédentes, les acides nucléiques, les ligands, les récepteurs ou leurs dérivés étant pourvus d'une étiquette détectable.

9. Procédé selon la revendication 8, l'étiquette étant choisie parmi le groupe des radio-isotopes, des isotopes stables, des enzymes, des liaisons immunoréactives, des produits chimiques fluorescents ou luminescents, des chromophores, des métaux ou des particules chargées.

10. Procédé selon au moins une des revendications précédentes, la solution des acides nucléiques englobant des acides nucléiques D et/ou des acides nucléiques L.

11. Procédé selon au moins une des revendications précédentes, une couche intermédiaire perméable aux ligands/récepteurs étant placée entre les surfaces.

12. Procédé selon la revendication 11, la couche intermédiaire étant choisie parmi le groupe d'un gel, d'une membrane, d'une polymère, d'une céramique et/ou d'un Capillar Tube Arrays.

13. Procédé selon au moins une des revendications précédentes, les acides nucléiques étant pourvus d'un groupe de tête chargé positivement.

14. Utilisation du procédé selon les revendications 2 ou 3 pour cloner des fragments génomiques de ADN, ADNc et ARN.

15. Utilisation selon la revendication 14 pour sub-cloner après la digestion de restriction.

16. Utilisation du procédé selon la revendication 1 pour amplifier le signal des ligands.

17. Utilisation du procédé selon la revendication 4 pour copier des gènes Chip.
